# EUROPÄISCHE PATENTSCHRIFT

(11) **EP 0 622 070 B1**
(45) Veröffentlichungstag und Bekanntmachung des Hinweises auf die Patenterteilung: **28.10.1998**
(21) Anmeldenummer: 94106050.1
(22) Anmeldetag: 19.04.1994
(51) Int. Cl.: A61K 7/42

(54) **Hautfärbende Pulvermischung**
Skindyeing powder mixture
Mélange pulvérulent pour la coloration de la peau

(30) Priorität: 29.04.1993 DE 4314083
(43) Veröffentlichungstag der Anmeldung: 02.11.1994
(73) Patentinhaber: MERCK PATENT GmbH, 64293 Darmstadt (DE)
(72) Erfinder: Kurz, Thekla, Dr., D-64846 Gross-Zimmern (DE); Hitzel, Sabine, D-64409 Messel (DE); Martin, Roland, Dr., D-69469 Weinheim (DE); Emmert, Ralf, Dr., D-64807 Dieburg (DE)

(56) Entgegenhaltungen:
- WO-A-92/19214
- DE-A- 1 492 438

## Beschreibung

Die Erfindung betrifft eine Pulvermischung, enthaltend Formaldehyd und/oder Ameisensaure freisetzende Verbindungen mit hautbräunenden Eigenschaften, welche ein Sulfitionen bildendes Agens enthalt.

Es ist seit langem bekannt, daß Verbindungen, welche eine Ketol-Gruppierung 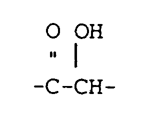 aufweisen, vorzugsweise Hydroxymethylketone, insbesondere Dihydroxyaceton, eine selbstbräunende Wirkung auf die menschliche Haut ausüben. Dieser selbstbräunende Effekt beruht im wesentlichen auf einer Maillard-Reaktion zwischen der Ketol-Gruppierung dieser Verbindungen und den Aminosäuren der Haut.

Die Farbtönung dieser Reaktion kann dabei durch Zusatz bestimmter Adjuvantien noch verstärkt werden (z.B. EP 0 425 324).

Weiterhin wird in der WO 91/12222 vorgeschlagen Dihydroxyaceton (DHA) zu stabilisieren, indem man es, zumindestens teilweise, in seiner dimeren Form mit einer wasserdichten Zusammensetzung unhüllt. Desweiteren wird dort vorgeschlagen, Formaldehyd-freisetzende Verbindungen zur Verhinderung des bakteriellen Abbaus von DHA einzusetzen.

Es wurde nun festgestellt, daß Verbindungen, wie z.B. DHA, welche eine Ketolgruppierung aufweisen, in den kosmetischen Zubereitungen langsam Formaldehyd und/oder Ameisensäure freisetzen. Dabei handelt es sich in der Regel nur um Spuren von Formaldehyd bzw. Ameisensäure, d.h. Formaldehyd-Anteile von wenigen ppm, in der Regel 20-100 ppm.

Aufgabe der vorliegenden Erfindung war es daher, pulverförmige Komponenten zu finden, die zu stabilen Mischungen verarbeitet werden, bei denen die Entstehung von Formaldehyd und Ameisensäure vollständig unterdrückt ist.

Überraschenderweise wurde gefunden, daß durch den Einsatz von Sulfitionen bildenden Agention die Bildung von Formaldehyd und Ameisensäure in solchen pulverförmigen Komponenten und in den daraus hergestellten Formulierungen vollständig unterdrückt werden kann.

Gegenstand der Erfindung ist somit eine Pulvermischung, enthaltend Formaldehyd und/oder Ameisensäure freisetzende Verbindungen mit hautbräunenden Eigenschaften, welche ein Sulfitionen bildendes Agens und gegebenenfalls einen Stabilisator enthält.

Gegenstand der Erfindung ist auch die Verwendung einer solchen Mischung bei der Herstellung von kosmetischen oder pharmazeutischen Zubereitungen.

Gegenstand der Erfindung ist weiterhin auch die Herstellung dieser Mischungen, wobei zumindest eine der Komponenten, insbesondere das Sulfitionen bildende Agens mit dem Stabilisator verrieben oder überzogen wird.

Bevorzugte Ausführungsformen sind;
a) Mischungen, wobei die Verbindung mit hautbräunenden Eigenschaften Dihydroxyaceton ist;
b) Mischungen, wobei das Sulfitionen bildende Agens ein Hydrogensulfit, Disulfit oder Dithionit, vorzugsweise ein Alkali- oder Erdalkalihydrogensulfit, Alkalidisulfit oder Alkalidithionit, insbesondere Natriumhydrogensulfit, Natriumbisulfit oder Natriumdithionit ist;
c) Mischungen, welche 45 bis 99, vorzugsweise 80 bis 90 Gew.-% einer Formaldehyd und/oder Ameisensäure freisetzenden Verbindung, 0,1 bis 50, vorzugsweise 1 bis 20 Gew.-% eines Sulfitionen bildenden Agens und 0,1 bis 5 Gew.-% eines Stabilisators jeweils bezogen auf die gesamte Mischung enthält;
d) Mischungen, wobei das Gewichtsverhältnis zwischen dem Sulfitionen bildenden Agens und der Formaldehyd und/oder Ameisensäure freisetzenden Verbindung größer als 0,01 ist;
e) Mischungen, wobei als Stabilisator ein Stearat, insbesondere Magnesiumstearat, enthalten ist.
f) Mischungen, wobei als Stabilisator ein filmbildendes Agens enthalten ist.
g) Mischungen, wobei als Stabilisator ein Cellulosederivat, insbesondere Ethylcellulose oder Nitrocellulose, enthalten ist.
h) Mischungen, wobei als Stabilisator ein höherer Alkohol wie z.B. Cetylalkohol, ein Paraffin oder ein anderes übliches polymeres Hüllmaterial enthalten ist.

Bevorzugte Verbindungen mit hautbräunenden Eigenschaften sind solche mit einer Ketol-Gruppierung; 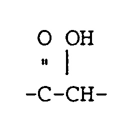 insbesondere Dihydroxyaceton.

Bevorzugte Sulfitionen bildende Agentien sind Metallsulfite, -disulfite bzw. -hydrogensulfite und Metalldithionite, wie z.B. Natriumhydrogensulfit, Calciumhydrogensulfit, Natriumsulfit, Kaliumsulfit, Natrium- oder Zinkdithionit sowie Natriumdisulfit.

Die erfindungsgemäße Mischung wird vorzugsweise eingesetzt bei der Herstellung von kosmetischen Zubereitungen als Mittel zur Bräunung menschlicher Epidermis. Solch ein Mittel liegt in verschiedenen, für diesen Typ üblicherweise verwendeten Formen vor. So kann es insbesondere in Form öliger oder ölig-alkoholischer Lotionen, Emulsionen, wie als Creme oder als Milch in Form ölig-alkoholischer, ölig-wäßriger oder wäßrig-alkoholischer Gele oder als feste Stifte vorliegen oder als Aerosol konditioniert sein.

Es kann kosmetische Adjuvanzien enthalten, welche in dieser Art von Mittel üblicherweise verwendet werden, wie z.B. Verdickungsmittel, weichmachende Mittel, Befeuchtungsmittel, grenzflächenaktive Mittel, Fungicide, Bakterizide, Konservierungsmittel, insbesondere 4-Hydroxybenzoesäurealkylester, Mittel gegen Schaumbildung, Parfums, Wachse, Lanolin, Treibmittel, Farbstoffe und/oder Pigmente, welche das Mittel selbst färben, UV-Filter zum Schutz vor UV-A und/oder UV-B-Strahlen und andere in der Kosmetik gewöhnlich verwendete Ingredienzien.

Wesentlich ist, daß die erfindungsgemäßen Zubereitungen weder Formaldehyd noch Formaldehyd-freisetzende Verbindungen als Bakterizide enthalten.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform ist eine Emulsion, welche als Creme oder Milch vorliegt und außer der hautbräunenden Verbindung Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin; Fettsäureestern, insbesondere Triglyceriden von Fettsäuren, oder ölig-alkoholische Lotionen auf Basis eines Niedrigalkohols, wie Ethanol, oder eines Glycols, wie Propylenglykol und/oder eines Polyols, wie Glycerin, und Ölen, Wachsen und Fettsäureestern, wie Triglyceriden von Fettsäuren, dar.

Das kosmetische Mittel kann auch als alkoholisches oder wäßriges Gel vorliegen, welches einen oder mehrere Niedrigalkohole oder -polyole, wie Ethanol, Propylenglycol oder Glycerin, und ein Verdickungsmittel, wie Kieselerde umfaßt.

Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäureestern, Lanolin und anderen Fettkörpern.

Geeignete Mittel sind auch kosmetische Sonnenschutzmittel, die mindestens einen UVB- und/oder UVA-Filter umfassen.

Geeignete UV-Filter sind zum Beispiel Derivate der Zimtsäure, Benzylidencampher und seine Derivate, p-Aminobenzoesäure und seine Derivate, Salizylsäurederivate, Benzophenonderivate und Dibenzoylmethanderivate. Diese UV-Filter sind, in der Regel, zu 0,2 bis 10 Gew.-% in den Zubereitungen enthalten.

Ist ein Mittel als Aerosol konditioniert, verwendet man in der Regel die üblichen Treibmittel, wie Alkane, Fluoralkane und Chlorfluoralkane.

Die Zubereitung kann gegebenenfalls Verdickungsmittel enthalten:

Zum Verdicken können die dem Fachmann geläufigen Verdickungsmittel oder Gelbildner verwendet werden, wie z.B. Guargummi, Heterobiopolysaccharide, Xanthangummi, Skleroglucane, Derivate der Cellulose, wie z.B. Methylcellulose, Hydroxyethylcellulose, Hydroxymethylpropylcellulose, Alkalisalze der Carboxymethylcellulose und Polyacrylsäuren.

Die Herstellung der erfindungsgemäßen Mischungen kann auf verschiedene, dem Fachmann an sich geläufige Methoden erfolgen. Überraschenderweise hat sich nämlich gezeigt, daß zur Erzielung einer stabilisierenden Wirkung lediglich dafür gesorgt werden muß, daß zwischen den einzelnen Pulverkomponenten kein allzu inniger Kontakt besteht.

Als Stabilisatoren geeignet sind daher z.B. übliche Gleitmittel wie z.B. Stearate, mit denen zumindest eine der Pulverkomponenten verrieben oder vermahlen wird. Offenbar reicht die dabei erzielte zumindest partielle Bedeckung der Oberfläche der Pulverkomponente für die erforderliche Stabilisierung aus. Dieses Verreiben der Pulverkomponente mit einem Gleitmittel kann in den üblichen, dem Fachmann geläufigen Vorrichtungen und Mischern durchgeführt werden.

Zur Stabilisierung können aber auch alle üblichen filmbildenden Agentien eingesetzt werden wie z.B. Cellulosederivate, höhere Alkohole, Paraffine und Polymere. Diese werden üblicherweise in einem geeigneten Lösungsmittel gelöst. Durch Anteigen der Pulverkomponente mit dieser Lösung und anschließendes Trocknen und Sieben erhält man ein Granulat, das dann mit der zweiten Komponente gemischt werden kann. In gleicher Weise kann aber auch die Pulverkomponente in einem Mischer mit der Lösung des Filmbildners besprüht und getrocknet werden oder es können andere dem Fachmann geläufige Methoden angewendet werden.

Wichtig ist, daß zumindest eine der Pulverkomponenten mit dem Stabilisator behandelt wird. Welche der beiden Komponenten dies ist, kann der Fachmann nach praktischen Gesichtspunkten enstscheiden, z.B. danach welche Komponente sich in üblichen Mischern oder Granuliereinrichtungen leichter behandeln läßt. Selbstverständlich können aber auch beide Pulverkomponenten behandelt werden.

Auch ohne weitere Ausführungen wird davon ausgegangen, daß ein Fachmann die obige Beschreibung in weitestem Umfang nutzen kann. Die nachfolgenden Beispiele sind deswegen lediglich als beschreibende, keineswegs als in irgendeiner Weise limitierende Offenbarung aufzufassen.

Die vollständige Offenbarung aller vor- und nachstehenden Anmeldungen, Patente und Veröffentlichungen, sind durch Bezugnahme in diese Anmeldung eingeführt.

### Beispiel 1

In einem Mischer werden 10 g Natriumsulfit intensiv mit 0,5 g Magnesiumstearat verrieben und anschließend mit 89,5 g Dihydroxyaceton gemischt. Man erhält eine lagerstabile Pulvermischung, die unmittelbar zur Herstellung von kosmetischen Zubereitungen eingesetzt werden kann.

### Beispiel 2

Man verfahrt analog Beispiel 1, setzt jedoch anstelle Natriumsulfit Natriumdisulfit ein.

### Beispiele 3 und 4

Man verfahrt analog Beispiel 1 und 2, setzt jedoch Calciumstearat anstelle von Magnesiumstearat ein.

### Beispiel 5

98 g Dihydroxyaceton werden mit einer Lösung von 2 g Ethylcellulose in 50 ml Ethanol angeteigt, getrocknet und gesiebt und danach mit 10 g Natriumsulfit gemischt.

### Beispiel 6

Man verfährt analog Beispiel 5, setzt jedoch Natriumdisulfit anstelle von Natriumsulfit ein.

### Beispiele 7-12

Man verfahrt analog den Beispielen 5 bzw. 6, setzt jedoch Nitrocellulose, Cetylalkohol bzw. Paraffin anstelle von Ethylcellulose ein.

| Anwendungsbeispiel A Selbstbräunungscreme (O/W) | | |
|---|---|---|
| | | % |
| A | Emulgator E 2155 (2) | 8,00 |
| | Paraffinöl flüssig (Art.-Nr. 7162) (1) | 12,00 |
| | Paraffin schüttfähig (Art.-Nr. 7158) (1) | 2,00 |
| | Miglyol 812 (3) | 3,00 |
| | Isopropylmyristat (4) | 2,00 |
| B | Propandiol-1,2 (Art.-Nr. 7478) (1) | 4,00 |
| | Karion F flüssig (Art.-Nr. 2993) (1) | 2,00 |
| | Konservierungsmittel (1) | q.s. |
| | Wasser, demineralisiert | ad 100,00 |
| C | DHA-Pulver hergestellt nach Beispiel 1 (1) | 5,50 |
| | Wasser, demineralisiert | 2,00 |

### Herstellung:

Phase A auf 75 °C, Phase B auf 80 °C erhitzen. Phase B langsam in Phase A einrühren. Unter Rühren abkühlen, bei 40 °C Phase C zugeben.

### Bemerkung:

Muster enthalten als Konservierungsmittel
0,05 % Propyl-4-hydroxybenzoat (Merck-Art.-Nr. 7427)
0,15 % Methyl-4-hydroxybenzoat (Merck-Art.-Nr. 6757)

### Bezugsquellen:

(1) E. Merck, Darmstadt
(2) Th. Goldschmidt, Essen
(3) Hüls Troisdorf AG, Witten
(4) Henkel, Düsseldorf

| Anwendungsbeispiel B Selbstbräunungsmilch (O/W) | | |
|---|---|---|
| | | % |
| A | Arlatone 983 S (2) | 1,50 |
| | Arlatone 985 (2) | 2,20 |
| | Brij 76 (2) | 1,50 |
| | Paraffinöl flüssig (Art.-Nr. 7162) (1) | 5,00 |
| | Miglyol 812 (3) | 5,00 |
| B | Karion F flüssig (Art.-Nr. 2993) (1) | 2,50 |
| | Propandiol-1,2 (Art.-Nr. 7478) (1) | 2,50 |
| | Konservierungsmittel (1) | q.s. |
| | Wasser, demineralisiert | ad 100,00 |
| C | DHA-Pulver hergestellt nach Beispiel 2 (1) | 5,50 |
| | Wasser, demineralisiert | 14,50 |

### Herstellung:

Phase A auf 75 °C, Phase B auf 80 °C erhitzen. Phase B langsam in Phase A einrühren. Homogenisieren. Unter Rühren abkühlen, bei 40 °C Phase C zugeben.

### Bemerkung:

Viskosität 14.000 mPas (Brookfield RVT, Sp. C, 10 Upm) bei 23 °C

Muster enthalten als Konservierungsmittel
0,05 % Propyl-4-hydroxybenzoat (Merck-Art.-Nr. 7427)
0,15 % Methyl-4-hydroxybenzoat (Merck-Art.-Nr. 6757)

### Bezugsquellen:

(1) E. Merck, Darmstadt
(2) ICI, Essen
(3) Hüls Troisdorf AG, Witten

| Anwendungsbeispiel C Selbstbräunungsmilch (W/O) | | |
|---|---|---|
| | | % |
| A | Arlacel 481 (2) | 3,15 |
| | Arlacel 989 (2) | 3,85 |
| | Paraffin dünnflüssig (Art.-Nr. 7174) (1) | 16,00 |
| | Isopropylmyristat (3) | 3,50 |
| | Miglyol 812 (4) | 3,50 |
| B | Propandiol-1,2 (Art.-Nr. 7478) (1) | 3,50 |
| | Magnesiumsulfat-Heptahydrat (Art.-Nr. 5882) (1) | 0,70 |
| | Konservierungsmittel (1) | q.s. |
| | Wasser, demineralisiert | ad 100,00 |
| C | DHA-Pulver hergestellt nach Beispiel 1 (1) | 5,50 |

### Herstellung:

Die Phasen A und B auf 75 °C erhitzen. Um eine Wärmebelastung des DHA-Pulvers durch zu langes Erhitzen zu vermeiden, wird Phase C in Phase B gelöst und in Phase A eingerührt. Homogenisieren. Unter Rühren schnell auf 25 °C abkühlen.

### Bemerkung:

Muster enthalten als Konservierungsmittel
0,05 % Propyl-4-hydroxybenzoat (Merck-Art.-Nr. 7427)
0,15 % Methyl-4-hydroxybenzoat (Merck-Art.-Nr. 6757)

### Bezugsquellen:

(1) E. Merck, Darmstadt
(2) ICI, Essen
(3) Henkel, Düsseldorf
(4) Hüls Troisdorf AG, Witten

| Anwendungsbeispiel D Selbstbräunungscreme (O/W) | | |
|---|---|---|
| | | % |
| A | Lanette N (2) | 15,00 |
| | Cetylalkohol (Art.-Nr. 989) (1) | 2,00 |
| | Cetylpalmitat (Art.-Nr. 15419) (1) | 5,00 |
| | Lanolin Corona (3) | 0,50 |
| | Cetiol (2) | 5,00 |
| | Vaseline (4) | 3,00 |
| | Oxynex 2004 (Art.-Nr. 6940) (1) | 0,05 |
| B | Karion F flüssig (Art.-Nr. 2993) (1) | 3,00 |
| | Konservierungsmittel | q.s. |
| | Wasser, demineralisiert | ad 100,00 |
| C | DHA-Pulver hergestellt nach Beispiel 3 (1) | 5,50 |
| | Wasser, demineralisiert | 14,50 |

### Herstellung:

Phase A auf 75 °C, Phase B auf 80 °C erhitzen. Phase B langsam in Phase A einrühren. Homogenisieren. Unter Rühren abkühlen, bei 40 °C Phase C zugeben und gegebenenfalls parfümieren.

### Bemerkung:

Muster enthalten als Konservierungsmittel
0,05 % Propyl-4-hydroxybenzoat (Merck-Art.-Nr. 7427)
0,15 % Methyl-4-hydroxybenzoat (Merck-Art.-Nr. 6757)

### Bezugsquellen:

(1) E. Merck, Darmstadt
(2) Henkel, Düsseldorf
(3) Croda, Nettertal
(4) E. Wagner, Bremen

| Anwendungsbeispiel E Selbstbräunungscreme (O/W) | | |
|---|---|---|
| | | % |
| A | Arlacel 165 (2) | 6,60 |
| | Atlas G-1790 (2) | 3,60 |
| | Lanette O (3) | 3,00 |
| | Paraffinöl flüssig (Art.-Nr. 7162) (1) | 1,50 |
| | Isopropylmyristat (3) | 4,00 |
| | Abil AV 200 (4) | 1,00 |
| | Oxynex 2004 (Art.-Nr. 6940) (1) | 0,05 |
| B | Karion F flüssig (Art.-Nr. 2993) (1) | 6,00 |
| | Konservierungsmittel | q.s. |
| | Wasser, demineralisiert | ad 100,00 |
| C | DHA-Pulver hergestellt nach Beispiel 4 (1) | 5,50 |
| | Wasser, demineralisiert | 14,50 |

### Herstellung:

Phase A auf 75 °C, Phase B auf 80 °C erhitzen. Phase B langsam in Phase A einrühren. Homogenisieren. Unter Rühren abkühlen, bei 40 °C Phase C zugeben und gegebenenfalls parfümieren.

### Bemerkung:

Muster enthalten als Konservierungsmittel
0,05 % Propyl-4-hydroxybenzoat (Merck-Art.-Nr. 7427)
0,15 % Methyl-4-hydroxybenzoat (Merck-Art.-Nr. 6757)

### Bezugsquellen:

(1) E. Merck, Darmstadt
(2) ICI, Essen
(3) Henkel, Düsseldorf
(4) Th. Goldschmidt, Essen

| Anwendungsbeispiel F Selbstbräunungsgel (wässrig) | | |
|---|---|---|
| | | % |
| A | DHA-Pulver hergestellt nach Beispiel 1 (1) | 5,50 |
| | Karion F flüssig (Art.-Nr. 2993) (1) | 2,50 |
| | Propandiol-1,2 (Art.-Nr. 7478) (1) | 2,50 |
| | Konservierungsmittel | q.s. |
| | Wasser, demineralisiert | ad 100,00 |
| B | Natrosol 250 HHR (2) | 1,50 |
| | Wasser, demineralisiert | 60,00 |

### Herstellung:

Für Phase B wird das Natrosol langsam in den Wirbel des kräftig gerührten Wassers gegeben. Die Zugabe erfolgt langsam und gleichmäßig, so daß die Partikel sich im Wasser ohne Klumpenbildung verteilen können. Für Phase A wird das DHA-Pulver in das Wasser eingerührt und die restlichen Rohstoffe unter Rühren zugegeben. Diese Suspension wird in Phase B eingerührt und homogenisiert.

### Bemerkung:

leicht opakes Gel
Muster enthalten als Konservierungsmittel
0,20 % Methyl-4-hydroxybenzoat (Merck-Art.-Nr. 6757) Eine Lagerung bei Raumtemperatur wird empfohlen, um einen Viskositätsabfall ab etwa 40 °C zu vermeiden.

### Bezugsquellen:

(1) E. Merck, Darmstadt
(2) Aqualon, Düsseldorf
   Bei den in den Anwendungsbeispielen A bis F beschriebenen Formulierungen konnte nach längerer Lagerung selbst bei erhöhter Temperatur kein Formaldehyd nachgewiesen werden.

### Vergleichsbeispiel 1

Man stellt eine Anwendungsbeispiel A entsprechende Selbstbräunungscreme (O/W) her, worin das erfindungsgemäße DHA-Pulver ersetzt ist durch:
- a) DHA, rein: 5,00 g, oder
- b) DHA unhüllt entsprechend WO 91/12222: 5,00 g

Folgende Formaldehyd- und Ameisensäure-Konzentration treten nach 3monatiger Lagerung bei 40 °C auf:

| | HCHO (ppm) | HCOOH (ppm) |
|---|---|---|
| Anwendungsbeispiel A | n.n. | n.n. |
| a) | 70 | 3569 |
| b) | 70 | 5825 |

### Vergleichsbeispiel 2

Man stellt eine Anwendungsbeispiel C entsprechende Selbstbräunungsmilch (W/O) her, worin das erfindungsgemäße DHA-Pulver ersetzt ist durch:
- a) DHA, rein: 5,00 g, oder
- b) DHA umhüllt entsprechend WO 91/12222: 5,00 g

Folgende Formaldehyd- und Ameisensäure-Konzentration treten nach 3monatiger Lagerung bei 40 °C auf:

| | HCHO (ppm) | HCOOH (ppm) |
|---|---|---|
| Anwendungsbeispiel C | n.n. | n.n. |
| a) | 33 | 3438 |
| b) | 23 | 2365 |

## Patentansprüche

1. Pulvermischung, enthaltend eine Formaldehyd und/oder Ameisensäure freisetzende Verbindung mit hautbräunenden Eigenschaften, dadurch gekennzeichnet, daß sie ein Sulfitionen bildendes Agens und gegebenenfalls einen Stabilisator enthält.

2. Mischung nach Anspruch 1, dadurch gekennzeichnet, daß die Verbindung mit hautbräunenden Eigenschaften Dihydroxyaceton ist.

3. Mischung nach Anspruch 1 oder 2, dadurch gekennzeichnet, daß das Sulfitionen bildende Agens ein Hydrogensulfit, vorzugsweise ein Alkali- oder Erdalkalihydrogensulfit, Disulfit, vorzugsweise ein Alkalidisulfit oder Dithionit ist.

4. Mischung nach Anspruch 3, dadurch gekennzeichnet, daß das Sulfitionen bildende Agens Natriumhydrogensulfit, vorzugsweise ein Alkalidithionit, Natriumdisulfit oder Natriumdithionit ist.

5. Mischung nach einem der Ansprüche 1-4, dadurch gekennzeichnet, daß als Stabilisator ein Stearat, ein Cellulosederivat oder ein Polymer enthalten ist.

6. Mischung nach einem der Ansprüche 1-5, dadurch gekennzeichnet, daß sie 45-99 Gew.-%, vorzugsweise 80 bis 90 Gew.-%, der Verbindung mit hautbräunenden Eigenschaften, 0,1 bis 50 Gew.-%, vorzugsweise 1-20 Gew.-%, des Sulfitionen bildenden Agens und 0,1 bis 5 Gew.-% des Stabilisators enthält.

7. Verwendung einer Mischung nach einem der Ansprüche 1-6 bei der Herstellung von kosmetischen oder pharmazeutischen Zubereitungen.

8. Verfahren zur Herstellung von Pulvermischungen nach Anspruch 1, dadurch gekennzeichnet, daß zumindest eine der Komponenten, vorzugsweise das Sulfitionen bildende Agens mit dem Stabilisator verrieben, vermahlen oder überzogen wird.

9. Verfahren nach Anspruch 8, dadurch gekennzeichnet, daß zumindest eine der Komponenten mit einem üblichen Gleitmittel verrieben oder vermahlen wird.

10. Verfahren nach Anspruch 9, dadurch gekennzeichnet, daß zumindest eine der Komponenten mit einem üblichen Filmbildner oder Polymeren überzogen wird.

## Claims

1. Powder mixture containing a formaldehyde- and/or formic acid-releasing compound which has skin-tanning properties, characterized in that the powder mixture contains an agent which forms sulphite ions and, if appropriate, stabilizers.

2. Mixture according to Claim 1, characterized in that the compound which has skin-tanning properties is dihydroxyacetone.

3. Mixture according to Claim 1 or 2, characterized in that the agent which forms sulphite ions is a hydrogen sulphite, preferably an alkali metal hydrogen sulphite or an alkaline earth metal hydrogen sulphite, disulphite, preferably an alkali metal disulphite, or dithionite.

4. Mixture according to Claim 3, characterized in that the agent which forms sulphite ions is sodium hydrogen sulphite, preferably an alkali metal dithionite, sodium disulphite or sodium dithionite.

5. Mixture according to one of Claims 1-4, characterized in that it contains, as stabilizer, a stearate, a cellulose derivative or a polymer.

6. Mixture according to one of Claims 1-5, characterized in that it contains 45-99% by weight, preferably 80 to 90% by weight, of the compound having skin-tanning properties, 0.1 to 50% by weight, preferably 1-20% by weight, of the agent which forms sulphite ions, and 0.1 to 5% by weight of the stabilizer.

7. Use of a mixture according to one of Claims 1-6 in the manufacture of cosmetic or pharmaceutical preparations.

8. Process for manufacturing powder mixtures according to Claim 1, characterized in that at least one of the components, preferably the agent which forms sulphite ions, is triturated, ground or coated with the stabilizer.

9. Process according to Claim 8, characterized in that at least one of the components is triturated or ground with a conventional lubricant.

10. Process according to Claim 9, characterized in that at least one of the components is coated with a conventional film-forming agent or polymer.

## Revendications

1. Mélange en poudre comportant un composé qui libère du formaldéhyde et/ou de l'acide formique et qui possède des propriétés pour faire bronzer la peau, caractérisé en ce qu'il comporte un agent formant des ions sulfites et, le cas échéant, un agent stabilisant.

2. Mélange selon la revendication 1, caractérisé en ce que le composé possédant des propriétés pour faire bronzer la peau est la dihydroxyacétone.

3. Mélange selon la revendication 1 ou 2, caractérisé en ce que l'agent formant des ions sulfites est un hydrogénosulfite, de préférence un hydrogenosulfite alcalin ou alcalino-terreux, un bisulfite de préférence un bisulfite alcalin, ou un dithionite.

4. Mélange selon la revendication 3, caractérisé en ce que l'agent formant des ions sulfites est l'hydrogénosulfite de sodium, de préférence un dithionite alcalin, le bisulfite de sodium ou le dithionite de sodium.

5. Mélange selon une des revendications 1 à 4, caractérisé en ce qu'il comporte comme agent stabilisant un stéarate, un dérivé de la cellulose ou un polymère.

6. Mélange selon une des revendications 1 à 5, caractérisé en ce qu'il comporte de 45 à 99 % en poids, de préférence de 80 à 90 % en poids, du composé possédant des propriétés pour faire bronzer la peau, de 0,1 à 50 % en poids, de préférence de 1 à 20 % en poids, de l'agent formant des ions sulfites et de 0,1 à 5 % en poids de l'agent stabilisant.

7. Utilisation d'un mélange selon une des revendications 1 à 6, lors de la production de préparations cosmétiques ou pharmaceutiques.

8. Procédé pour produire des mélanges en poudre selon la revendication 1, caractérisé en ce qu'au moins un des composants, de préférence l'agent formant des ions sulfites, est malaxé ou broyé avec l'agent stabilisant, ou recouvert par celui-ci.

9. Procédé selon la revendication 8, caractérisé en ce qu'au moins un des composants est malaxé ou broyé avec un lubrifiant habituel.

10. Procédé selon la revendication 9, caractérisé en ce qu'au moins un des composants est recouvert par au moins un agent filmogène, ou polymère, habituel.
